# EUROPEAN PATENT APPLICATION

(11) **EP 4 803 499 A1**
(43) Date of publication of application: **09.09.2026**
(21) Application number: 26162005.8
(22) Date of filing: 03.03.2026
(51) Int. Cl.: C07C 29/151, C07C 31/04

(54) **METHANOL PRODUCTION SYSTEM THAT REUSES UNREACTED GAS FOR EFFICIENT METHANOL PRODUCTION**

(30) Priority: 04.03.2025 KR 20250027710
(71) Applicant: Doosan Enerbility Co., Ltd., Seongsan-gu Changwon-si, Gyeongsangnam-do 51711 (KR)
(72) Inventor: Kim, Bong Keun, 16814 Yongin-si, Gyeonggi-do (KR); Bae, Seong Hee, 18475 Hwaseong-si, Gyeonggi-do (KR); Kim, Hyun Min, 16868 Yongin-si, Gyeonggi-do (KR); Nam, Gyeong Mo, 16821 Yongin-si, Gyeonggi-do (KR); Kim, You Seok, 16923 Yongin-si, Gyeonggi-do (KR); Sohn, Geun, 13607 Seongnam-si, Gyeonggi-do (KR)
(74) Representative: BCKIP Part mbB

(57) **Abstract**

A methanol production system capable of improving methanol production efficiency is provided. The methanol production system includes a waste gas reformer configured to reform a waste gas comprising a hydrocarbon (CxHy) gas and carbon dioxide to generate synthesis gas containing hydrogen, carbon monoxide, and carbon dioxide; a methanol production reactor including a first reaction zone to which a portion of the synthesis gas is supplied to generate methanol in the presence of a first catalyst, and a second reaction zone to which a remaining portion of the synthesis gas is supplied to generate methanol in the presence of a second catalyst; a condenser configured to condense methanol and water from gas discharged from the methanol production reactor; and a gas separator configured to separate a recycle gas comprising hydrogen, or a mixture of hydrogen and carbon monoxide, from gas discharged from the condenser after methanol and water are condensed and separated.

## Description

This application claims priority to Korea Patent Application No. 10-2025-0027710, filed on March 04, 2025.

### FIELD

Apparatuses and methods consistent with exemplary embodiments relate to a methanol production system, and more particularly, to a methanol production system configured to increase methanol production efficiency by utilizing synthesis gas including carbon monoxide, hydrogen, and high-concentration carbon dioxide, and by separating and recycling hydrogen following the methanol production reaction.

### BACKGROUND

In a related art, synthesis gas containing carbon monoxide and hydrogen is supplied to a methanol production reactor, and methanol is produced in the reactor via a catalytic reaction of carbon monoxide and hydrogen.

In the related art systems, carbon dioxide levels in the synthesis gas supplied to the reactor have to be restricted to a low level, for example, below 5%. When carbon dioxide is contained in the synthesis gas, an additional process is necessitated to separate and remove the carbon dioxide before the synthesis gas is introduced into the reactor. In other words, only carbon monoxide and hydrogen are selectively supplied from the synthesis gas to the reactor.

### SUMMARY

Aspects of one or more exemplary embodiments provide a methanol production system capable of improving methanol production efficiency by utilizing synthesis gas including carbon monoxide, hydrogen, and high-concentration carbon dioxide, and by separating and recycling hydrogen following the methanol production reaction.

Additional aspects will be set forth in part in the description which follows and, in part, will become apparent from the description, or may be learned by practice of the exemplary embodiments.

According to an aspect of an exemplary embodiment, there is provided a methanol production system including: a waste gas reformer configured to reform a waste gas comprising a hydrocarbon (CxHy) gas and carbon dioxide to generate synthesis gas containing hydrogen, carbon monoxide, and carbon dioxide; a methanol production reactor including a first reaction zone to which a portion of the synthesis gas is supplied to generate methanol in the presence of a first catalyst, and a second reaction zone to which a remaining portion of the synthesis gas is supplied to generate methanol in the presence of a second catalyst; a condenser configured to condense methanol and water from gas discharged from the methanol production reactor; and a gas separator configured to separate a recycle gas comprising hydrogen, or a mixture of hydrogen and carbon monoxide, from gas discharged from the condenser after methanol and water are condensed and separated.

An unreacted gas from the first reaction zone may be supplied to the second reaction zone concurrently with the remaining portion of the synthesis gas.

The gas separator may include a pressure swing adsorption (PSA) apparatus or a membrane configured to separate hydrogen, and the hydrogen separated in the gas separator may be recycled for methanol production as the recycle gas.

The gas separator may include a membrane configured to separate carbon dioxide, and hydrogen and carbon monoxide remaining after the separation of carbon dioxide by the gas separator may be recycled for methanol production as the recycle gas.

The recycle gas separated by the gas separator may be merged with the synthesis gas supplied to the first reaction zone.

A molar ratio of (H₂-CO₂)/(CO+CO₂) of the synthesis gas supplied to the first reaction zone after merging with the recycle gas may be maintained within a range of approximately 1.5 to 2.5.

The methanol production system may further include at least one first compressor configured to compress the synthesis gas supplied to the first reaction zone and the second reaction zone, and wherein the first compressor may be configured to pressurize the synthesis gas to a pressure within a range of approximately 20 bar to 100 bar.

The methanol production system may further include a second compressor configured to compress the recycle gas separated by the gas separator and merge the compressed recycle gas with the synthesis gas supplied to the first reaction zone.

The methanol production system may further include at least one heat exchanger configured to heat the synthesis gas supplied to the first reaction zone and the second reaction zone, and wherein the synthesis gas may be heated to a temperature within a range of approximately 150 to 300°C by the heat exchanger.

The heat exchanger may use the gas discharged from the methanol production reactor as a heat source.

A portion of the gas discharged from the condenser after methanol and water are condensed and separated may be branched and supplied to the gas separator, and the methanol production system may further include a flow control valve configured to control a flow rate of the gas branched and supplied to the gas separator.

The methanol production reactor may be configured as a single reactor in which the first reaction zone and the second reaction zone are disposed.

The methanol production system may further include a distillation column in fluid communication with the condenser, and the distillation column may be configured to receive the methanol and water condensed by the condenser to separate the methanol and water to produce high-concentration methanol.

The waste gas supplied to the waste gas reformer may have a molar ratio of methane to carbon dioxide of 7:3 or less.

The synthesis gas generated by the waste gas reformer may include 0% to 70% hydrogen, 0% to 50% carbon monoxide, 0% to 20% carbon dioxide, and 5% or less methane.

According to one or more exemplary embodiments, the system is configured to produce methanol using synthesis gas containing carbon monoxide, hydrogen, and high-concentration carbon dioxide. Because both carbon monoxide and carbon dioxide serve as reactive species with hydrogen to produce methanol within a single reactor, methanol can be produced under high carbon dioxide concentration conditions without pre-reaction separation or removal of carbon dioxide from synthesis gas generated by reforming waste gas such as biogas or pyrolysis gas.

In addition, the system is configured to separate hydrogen, or a mixture of hydrogen and carbon monoxide, as a recycle gas following the methanol production reaction. By recycling the recycle gas for methanol production, methanol production efficiency can be improved. Specifically, by using the recycle gas, a methanol synthesis ratio R((H₂-CO₂)/(CO+CO₂)) of the synthesis gas supplied to the first reaction zone can be adjusted within a range of 1.5 to 2.5, thereby maximizing methanol production efficiency.

### BRIEF DESCRIPTION OF THE DRAWINGS

The above and other will be more clearly understood from the following description of the exemplary embodiments with reference to the accompanying drawings, in which:
FIG. 1 is a schematic diagram schematically illustrating a methanol production system according to a first exemplary embodiment; and
FIG. 2 is a schematic diagram schematically illustrating a methanol production system according to a second exemplary embodiment.

### DETAILED DESCRIPTION

Various modifications and various embodiments will be described with reference to accompanying drawings so that those skilled in the art can easily carry out the disclosure. It should be understood, however, that the various embodiments are not for limiting the scope of the disclosure to the specific embodiments, but they should be interpreted to include all modifications, equivalents or alternatives of the embodiments included within the spirit and scope disclosed herein.

The terms described below are for the purpose of describing specific embodiments only, and are not intended to limit the scope of the present disclosure. As used herein, the singular forms "a", "an" and "the" are intended to include the plural forms as well, unless the context clearly indicates otherwise. In the disclosure, terms such as "comprises", "includes", or "have/has" should be construed as designating that there are such features, integers, steps, operations, components, parts, and/or combinations thereof, and do not exclude the presence or possibility of adding of one or more of other features, integers, steps, operations, components, parts, and/or combinations thereof.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

Terms such as "first," "second," and so on may be used to describe a variety of elements, but the elements should not be limited by these terms. The terms are used simply to distinguish one element from other elements. The use of such ordinal numbers should not be construed as limiting the meaning of the term. For example, the components associated with such an ordinal number should not be limited in the order of use, placement order, or the like. If necessary, each ordinal number may be used interchangeably.

The terms, such as 'part' or 'module', etc., should be understood as a unit that performs at least one function or operation and that may be embodied as hardware, software, or a combination thereof. With respect to an element described as a "unit" or "module", two or more elements may be combined into a single element, or a single element may be divided into two or more elements according to the subdivided functions. Also, each element described below may additionally perform some or all of functions performed by other elements, in addition to its main functions, and some of the main functions of each element may be performed entirely by other components.

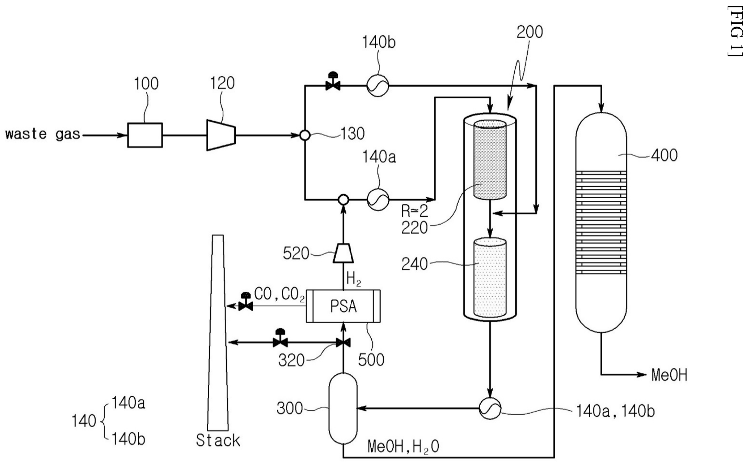

Hereinafter, a methanol production system according to a first exemplary embodiment will be described in detail with reference to FIG. 1.

Referring to FIG. 1, the methanol production system includes a waste gas reformer 100, a first compressor 120, a heat exchanger 140, a methanol production reactor 200, a condenser 300, a distillation column 400, a gas separator 500, and a second compressor 520.

The waste gas reformer 100 reforms waste gas, which is a hydrocarbon (CxHy) gas containing carbon dioxide, to generate synthesis gas containing hydrogen, carbon monoxide, and carbon dioxide. The waste gas may comprise any gas containing carbon dioxide and hydrocarbons (CxHy), such as biogas or pyrolysis gas. For example, the waste gas introduced into the waste gas reformer 100 has a molar ratio of methane to carbon dioxide of approximately 7:3, or a lower methane molar ratio, such as 6.5:3.5.

More specifically, the waste gas reformer 100 directly reforms the waste gas into synthesis gas by sequentially passing the waste gas through a first reformer in which the waste gas is reacted and reformed at a first temperature, and a second reformer in which the waste gas is reacted and reformed at a second temperature higher than the first temperature. For example, when the waste gas is pyrolysis gas containing carbon dioxide and higher hydrocarbons having two or more carbon atoms, the higher hydrocarbons may react with steam in the first reformer to form methane, and the methane may react with steam in the second reformer to form synthesis gas. In another example, when the waste gas is biogas containing carbon dioxide and methane, the methane may react with steam in the first reformer to form synthesis gas, and the methane may react with carbon dioxide in the second reformer to form synthesis gas.

The synthesis gas generated by the waste gas reformer 100 includes carbon monoxide, carbon dioxide, and hydrogen. The synthesis gas generated in the waste gas reformer 100 includes approximately 0% to 70% hydrogen, 0% to 50% carbon monoxide, 0% to 20% carbon dioxide, and 5% or less methane. Specifically, a concentration of carbon dioxide in the synthesis gas may be at leas 5%, and a molar ratio of carbon monoxide to carbon dioxide may be approximately 1:1. For example, the synthesis gas composition may include 65% to 67% hydrogen (H₂), 15% carbon monoxide (CO), 15% to 17% carbon dioxide (CO₂), and 0.3% to 0.4% methane (CH₄).

The synthesis gas is supplied to the methanol production reactor 200, which is configured to produce methanol utilizing synthesis gas containing carbon monoxide, hydrogen, and high-concentration carbon dioxide. Accordingly, the system enables methanol production under high carbon dioxide concentration conditions without requiring an upstream separation or removal process to eliminate carbon dioxide from the synthesis gas prior to the reaction.

In the methanol production reactor 200, carbon monoxide and carbon dioxide are each reacted with hydrogen to produce methanol. Specifically, the methanol production reactor 200 includes a first reaction zone 220 and a second reaction zone 240 disposed therein. A portion of the synthesis gas is supplied to the first reaction zone 220 and reacted in the presence of a first catalyst to generate methanol. Subsequently, a remaining portion of the synthesis gas is supplied to the second reaction zone 240 and reacted in the presence of a second catalyst to generate methanol. As such, the methanol production reactor 200 may be configured as a single reactor containing both the first reaction zone 220 and the second reaction zone 240.

In addition, an unreacted gas that is unreacted in the first reaction zone 220 is supplied to the second reaction zone 240 together with the remaining portion of the synthesis gas.

In the first reaction zone 220, carbon monoxide and hydrogen mainly react in the presence of the first catalyst to produce methanol primarily according to Chemical Formula (1), and in the second reaction zone 240, carbon dioxide and hydrogen react in the presence of the second catalyst to produce methanol primarily according to Chemical Formula (2).

Chemical Formula (1) - CO + 2H₂ → CH₃OH

Chemical Formula (2) - CO₂ + 3H₂ → CH₃OH + H₂O

In one or more exemplary embodiments, the synthesis gas generated in the waste gas reformer 100 is divided at a branching point 130. The branching point 130 may include a gas composition regulator configured to distribute the synthesis gas such that a first stream supplied to the first reaction zone 220 is enriched in carbon monoxide, while a second stream supplied to the second reaction zone 240 is enriched in carbon dioxide. Accordingly, the first reaction zone 220 is configured to primarily facilitate the reaction between carbon monoxide and hydrogen, and the second reaction zone 240 is configured to primarily facilitate the reaction between carbon dioxide and hydrogen.

To adjust the synthesis gas discharged from the waste gas reformer 100 to a pressure and temperature suitable for methanol production, at least one first compressor 120 and at least one heat exchanger 140 are arranged between the waste gas reformer 100 and the methanol production reactor 200.

The first compressor 120 is configured to compress the synthesis gas supplied to the first reaction zone 220 and the second reaction zone 240. In one embodiment, the first compressor 120 is disposed upstream of the branching point 130. In this configuration, the synthesis gas discharged from the waste gas reformer 100 is compressed by the first compressor 120 prior to being divided at the branching point 130 for respective supply to the first reaction zone 220 and the second reaction zone 240. The synthesis gas may be pressurized by the first compressor 120 to a pressure ranging from 20 bar to 100 bar. However, it is understood that this is only an example and other exemplary embodiments are not limited thereto. In an alternative embodiment, the system may comprise a plurality of compressors, such that the synthesis gas branched at the branching point 130 is compressed by separate compressors dedicated to the first reaction zone 220 and the second reaction zone 240, respectively.

The heat exchanger 140 is configured to heat the synthesis gas supplied to the first reaction zone 220 and the second reaction zone 240. In one or more exemplary embodiments, a plurality of heat exchangers 140 are disposed downstream of the branching point 130. Specifically, a first heat exchanger 140a is positioned to heat the portion of the synthesis gas supplied to the first reaction zone 220, and a second heat exchanger 140b is positioned to heat the portion of the synthesis gas supplied to the second reaction zone 240. The synthesis gas is preferably heated to a temperature ranging from 150°C to 300°C by the heat exchangers 140a and 140b. However, the present disclosure is not limited thereto, and in an alternative embodiment, a single heat exchanger 140 may be disposed upstream of the branching point 130 to heat the synthesis gas prior to division.

In FIG. 1, the heat exchanger 140a is schematically depicted in two separate positions to represent a single heat exchanger 140a. Similarly, the heat exchanger 140b is schematically depicted in two separate positions to represent a single heat exchanger 140b.

In one exemplary embodiment, the synthesis gas supplied to the first reaction zone 220 and the second reaction zone 240 is maintained at a pressure of approximately 70 bar and a temperature of approximately250°C.

A condenser 300 is disposed downstream of the methanol production reactor (200) and is configured to condense methanol and water from gas discharged from the second reaction zone 240. The methanol and water condensed in the condenser 300 are subsequently delivered to a distillation column 400, which is configured to separate the methanol and water to produce high-concentration methanol.

In one or more exemplary embodiments, the heat exchanger 140 may use the gas discharged from the methanol production reactor 200 as a heat source. Accordingly, the gas discharged from the second reaction zone 240 may be supplied to the condenser 300 after its temperature is lowered while heating the synthesis gas, thereby improving the efficiency of the condenser 300.

The gas discharged from the condenser 300, following the condensation and separation of methanol and water, includes hydrogen, carbon monoxide, and carbon dioxide unreacted in the methanol production reactor 200. The gas discharged from the condenser 300 may be subsequently directed to a stack for discharge from the system.

The gas separator 500 is configured to separate hydrogen, or a mixture of hydrogen and carbon monoxide, as a recycle gas from the gas discharged from the condenser 300. While the entire volume of the gas discharged from the condenser 300 may be supplied to the gas separator 500, in one or more exemplary embodiments, a portion of the gas discharged from the condenser 300 after methanol and water are condensed and separated is branched and supplied to the gas separator 500.

In this case, a flow control valve 320 may be disposed within the branched line to control a flow rate of the gas supplied to the gas separator 500. The flow control valve 320 is configured to adjust the volume of the branched gas stream to provide an optimal gas flow rate to the gas separator 500.

For example, the gas separator 500 comprises a pressure swing adsorption (PSA) apparatus configured to isolate hydrogen. However, the present disclosure is not limited thereto, and the gas separator 500 may alternatively comprise a hydrogen separation membrane or other suitable gas-selective separation structures. Accordingly, hydrogen separated by the gas separator 500 may be returned to the system as a recycle gas for methanol production. Conversely, the remaining components, such as carbon monoxide and carbon dioxide, that are rejected by the gas separator 500 are discharged to a stack.

The recycle gas separated in the gas separator 500 is merged with the synthesis gas supplied to the first reaction zone 220. Specifically, the recycle gas is merged with the synthesis gas downstream of the branching point 130 and upstream of the heat exchanger 140a, such that the synthesis gas merged with the recycle gas is supplied to the heat exchanger 140a.

A second compressor 520 may be further provided to compress the recycle gas separated in the gas separator 500 and merge the compressed recycle gas with the synthesis gas supplied to the first reaction zone 220. Because the pressure of the recycle gas is reduced during the separation process, the second compressor 520 is configured to pressurize the recycle gas to a pressure substantially equal to the pressure of the synthesis gas at the point of merging.

In one or more exemplary embodiments, it is preferable that a methanol synthesis ratio R = (H₂-CO₂)/(CO+CO₂) of the synthesis gas supplied to the first reaction zone 220 after merging with the recycle gas is within a range of approximately 1.5 to 2.5. Accordingly, after the methanol production reaction, hydrogen is used as a recycle gas to adjust the methanol synthesis ratio R of the synthesis gas supplied to the first reaction zone 220 to a range of 1.5 to 2.5, specifically to 2.0, thereby maximizing methanol production efficiency.

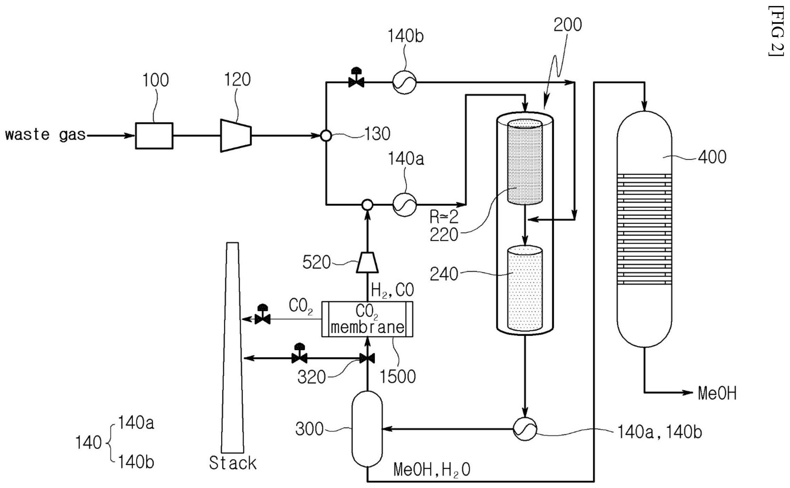

A methanol production system according to a second exemplary embodiment will be described with reference to FIG. 2.

Referring to FIG. 2, the methanol production system includes a waste gas reformer 100, a first compressor 120, a heat exchanger 140, a methanol production reactor 200, a condenser 300, a distillation column 400, a gas separator 1500, and a second compressor 520. The methanol production system of FIG. 2 is substantially similar to the methanol production system of FIG. 1 except for the configuration of the gas separator 1500.Accordingly, for the sake of brevity, only the features of the gas separator 1500 that differ from the first exemplary embodiment will be described.

In the present embodiment, the gas separator 1500 comprises a carbon dioxide separation membrane configured to selectively separate carbon dioxide. However, the present disclosure is not limited thereto, and the gas separator 1500 may comprise various other structural devices suitable for carbon dioxide isolation. In this configuration, carbon dioxide is separated and removed by the gas separator 1500, while the remaining hydrogen and carbon monoxide may be recycled for methanol production as a recycle gas. The carbon dioxide separated by the gas separator 1500 is subsequently discharged to a stack.

Similarly, the recycle gas separated by the gas separator 1500 is merged with the synthesis gas supplied to the first reaction zone 220.

While one or more exemplary embodiments have been described with reference to the accompanying drawings, it will be apparent to those skilled in the art that various variations and modifications may be made by adding, changing, or removing components without departing from the spirit and scope of the disclosure as defined in the claims, and these variations and modifications fall within the spirit and scope of the disclosure as defined in the appended claims. Therefore, the description of the exemplary embodiments should be construed in a descriptive sense and not to limit the scope of the claims, and many alternatives, modifications, and variations will be apparent to those skilled in the art.

## Claims

1. A methanol production system comprising:
a waste gas reformer configured to reform a waste gas comprising a hydrocarbon (CxHy) gas and carbon dioxide to generate synthesis gas containing hydrogen, carbon monoxide, and carbon dioxide;
a methanol production reactor including a first reaction zone to which a portion of the synthesis gas is supplied to generate methanol in the presence of a first catalyst, and a second reaction zone to which a remaining portion of the synthesis gas is supplied to generate methanol in the presence of a second catalyst;
a condenser configured to condense methanol and water from gas discharged from the methanol production reactor; and
a gas separator configured to separate a recycle gas comprising hydrogen, or a mixture of hydrogen and carbon monoxide, from gas discharged from the condenser after methanol and water are condensed and separated.

2. The methanol production system according to claim 1,
wherein an unreacted gas from the first reaction zone is supplied to the second reaction zone concurrently with the remaining portion of the synthesis gas.

3. The methanol production system according to claim 2,
wherein the gas separator comprises a pressure swing adsorption (PSA) apparatus or a membrane configured to separate hydrogen, and the hydrogen separated in the gas separator is recycled for methanol production as the recycle gas.

4. The methanol production system according to claim 2,
wherein the gas separator comprises a membrane configured to separate carbon dioxide, and hydrogen and carbon monoxide remaining after the separation of carbon dioxide by the gas separator are recycled for methanol production as the recycle gas.

5. The methanol production system according to claim 2,
wherein the recycle gas separated by the gas separator is merged with the synthesis gas supplied to the first reaction zone.

6. The methanol production system according to claim 5,
wherein a molar ratio of (H₂-CO₂)/(CO+CO₂) of the synthesis gas supplied to the first reaction zone after merging with the recycle gas is maintained within a range of approximately 1.5 to 2.5.

7. The methanol production system according to claim 5, further comprising:
at least one first compressor configured to compress the synthesis gas supplied to the first reaction zone and the second reaction zone, and
wherein the first compressor is configured to pressurize the synthesis gas to a pressure within a range of approximately 20 bar to 100 bar.

8. The methanol production system according to claim 7, further comprising:
a second compressor configured to compress the recycle gas separated by the gas separator and merge the compressed recycle gas with the synthesis gas supplied to the first reaction zone.

9. The methanol production system according to claim 5, further comprising:
at least one heat exchanger configured to heat the synthesis gas supplied to the first reaction zone and the second reaction zone, and
wherein the synthesis gas is heated to a temperature within a range of approximately 150 to 300°C by the heat exchanger.

10. The methanol production system according to claim 9,
wherein the heat exchanger uses the gas discharged from the methanol production reactor as a heat source.

11. The methanol production system according to claim 1,
wherein a portion of the gas discharged from the condenser after methanol and water are condensed and separated is branched and supplied to the gas separator, and
the methanol production system further comprising:
a flow control valve configured to control a flow rate of the gas branched and supplied to the gas separator.

12. The methanol production system according to claim 1,
wherein the methanol production reactor is configured as a single reactor in which the first reaction zone and the second reaction zone are disposed.

13. The methanol production system according to claim 1, further comprising:
a distillation column in fluid communication with the condenser, and
wherein the distillation column is configured to receive the methanol and water condensed by the condenser to separate the methanol and water to produce high-concentration methanol.

14. The methanol production system according to claim 1,
wherein the waste gas supplied to the waste gas reformer has a molar ratio of methane to carbon dioxide of 7:3 or less.

15. The methanol production system according to claim 1,
wherein the synthesis gas generated by the waste gas reformer includes 0% to 70% hydrogen, 0% to 50% carbon monoxide, 0% to 20% carbon dioxide, and 5% or less methane.
